# EUROPEAN PATENT SPECIFICATION

(11) **EP 2 827 952 B1**
(45) Date of publication and mention of the grant of the patent: **07.07.2021**
(21) Application number: 13764527.1
(22) Date of filing: 15.03.2013
(51) Int. Cl.: A61K 31/192, A61K 31/506, A61K 31/58, A61P 17/14, A61K 8/42, A61K 8/49, A61K 8/63, A61Q 7/00

(54) **COMPOSITION FOR STIMULATING HAIR GROWTH**
ZUSAMMENSETZUNG ZUR ANREGUNG DES HAARWUCHSES
COMPOSITION POUR STIMULER LA POUSSE DES CHEVEUX

(30) Priority: 21.03.2012 US 201261613829 P
(43) Date of publication of application: 28.01.2015
(73) Proprietor: The Trustees of The University of Pennsylvania, Philadelphia, PA 19104-6283 (US)
(72) Inventor: COTSARELIS, George, Berwyn, PA 19312 (US); FITZGERALD, Garret, Wayne, PA 19087 (US); GARZA, Luis, Baltimore, MD 21212 (US)
(74) Representative: Pearl Cohen Zedek Latzer Baratz UK LLP
(86) International application number: PCT/US2013/031825
(87) International publication number: WO 2013/142295

(56) References cited:
- WO-A1-2008/012511
- WO-A1-2012/078649
- WO-A2-2011/014588
- WO-A2-2011/085033
- US-A1- 2005 112 075
- US-A1- 2010 172 865
- US-A1- 2011 021 599
- GARZA L A ET AL: "Evidence that Prostaglandin D-2 contributes to development of Androgenetic Alopecia", JOURNAL OF INVESTIGATIVE DERMATOLOGY; 69TH ANNUAL MEETING OF THE SOCIETY OF INVESTIGATIVE DERMATOLOGY; MONTREAL, CANADA; MAY 06-09, 2009, NATURE PUBLISHING GROUP, US, vol. 129, no. Suppl. 1, April 2009 (2009-04), page S98, XP002581511, ISSN: 0022-202X
- GARZA L A ET AL: "The role of Prostaglandin D2 and its receptor DP-2 in promotion of Androgenetic Alopecia", JOURNAL OF INVESTIGATIVE DERMATOLOGY, vol. 130, no. Suppl. 1, April 2010 (2010-04), page S103, XP9188519, & ANNUAL MEETING OF THE SOCIETY-FOR-INVESTIGATIVE-DERMATOLOGY; ATLANTA, GA, USA; MAY 05 -08, 2010 ISSN: 0022-202X
- OHYAMA ET AL: "Management of hair loss diseases", DERMATOLOGICA SINICA, ELSEVIER, AMSTERDAM, NL, vol. 28, no. 4, December 2010 (2010-12), pages 139-145, XP027582578, ISSN: 1027-8117 [retrieved on 2010-12-01]
- ROY PETTIPHER ET AL: "Update on the Development of Antagonists of Chemoattractant Receptor-Homologous Molecule Expressed on Th2 Cells (CRTH2). From Lead Optimization to Clinical Proof-of-Concept in Asthma and Allergic Rhinitis", JOURNAL OF MEDICINAL CHEMISTRY, vol. 55, no. 7, 12 April 2012 (2012-04-12) , pages 2915-2931, XP055046242, ISSN: 0022-2623, DOI: 10.1021/jm2013997
- GARZA ET AL.: 'Prostaglandin D2 Inhibits Hair Growth and Is Elevated in Bald Scalp of Men with Androgenetic Alopecia' SCIENCE TRANSLATIONAL MEDICINE vol. 4, no. 126, 21 March 2012, pages 1 - 21, XP055096198
- DIAMANT ET AL: "Effect of multiple-dose setipiprant , a selective oral CRTH2 antagonist, on allergen-induced airway responses in allergic asthmatic patients", AMERICAN JOURNAL OF RESPIRATORY AND CRITICAL CARE MEDICINE, AMERICAN LUNG ASSOCIATION, NEW YORK, NY, US, vol. 185, 1 January 2012 (2012-01-01), XP009188573, ISSN: 1073-449X
- NORMAN ET AL: "A novel DP2 receptor antagonist (AM-461): a patent evaluation of WO2011085033", EXPERT OPINION ON THERAPEUTIC PATENTS, TAYLOR & FRANCIS, GB , vol. 21, no. 12 1 January 2011 (2011-01-01), pages 1931-1936, XP009523493, ISSN: 1354-3776, DOI: 10.1517/13543776.2011.636738 Retrieved from the Internet: URL:http://www.tandfonline.com/doi/full/10 .1517/13543776.2011.636738 [retrieved on 2011-11-15]
- Peter Norman: "DP 2 receptor antagonists in development", Expert Opinion on Investigational Drugs, vol. 19, no. 8, 18 August 2010 (2010-08-18), pages 947-961, XP055740809, UK ISSN: 1354-3784, DOI: 10.1517/13543784.2010.500019

## Description

### FIELD OF THE INVENTION

The invention relates to compositions for regulating hair growth. Specifically, the invention relates to regulating hair growth by regulating the activity one of the prostaglandin D2 (PGD2) receptors, DP-2 (GPR44).

### BACKGROUND OF THE INVENTION

Eighty percent of Caucasian men experience some degree of androgenetic alopecia (AGA) before age 70. Testosterone is necessary for AGA to develop, and a genetic susceptibility locus in the androgen receptor is present in a minority of men with AGA; however, additional factors contributing to this disorder remain unknown. Studies on AGA have the potential to yield insights into other androgen-mediated diseases, such as benign prostatic hypertrophy and prostate cancer. Current legitimate treatments for AGA include finasteride, minoxidil, and hair transplantation. Finasteride inhibits 5-a reductase 2 (SRD5A2), which converts testosterone to a more potent androgen, dihydrotestosterone. The active targets of minoxidil in AGA therapy have not been conclusively identified.

In AGA, large "terminal" hair follicles forming thick hair shafts miniaturize over time to small follicles that generate microscopic effete hairs. Follicle miniaturization is accompanied by a decrease in the duration of the growing phase of the follicle (anagen), which normally lasts several years to produce hair more than 1 m long, but which decreases to only days or weeks in AGA. This results in an increase in the percentage of resting (telogen) hair follicles containing microscopic hairs in bald scalp.

In addition to these intrinsic changes to the hair follicle, infiltrating lymphocytes and mast cells have been identified around the miniaturizing follicle, especially in the area of the stem cell-rich bulge area. Sebaceous glands, which attach to each follicle, hypertrophy in bald scalp. In balding scalp, the number of hair follicle stem cells remains intact, whereas the number of more actively proliferating progenitor cells markedly decreases. This indicates that balding scalp either lacks an activator or has an inhibitor of hair follicle growth.

US Patent Publication No. 2011/0021599 A1 describes methods and compositions for inhibiting or reducing hair loss (such as AGA or other types of alopecia), acne, rosacea, prostate cancer, and benign prostatic hypertrophy (BPH) by administering a compound or composition capable of decreasing prostaglandin D2 (PGD2) or a downstream pathway thereof, such as via topical administration of a DP-2 receptor antagonist.

US Patent Publication No. 2005/0112075 A1 describes methods of reducing unwanted hair growth by topical application of a prostaglandin DP-receptor agonist, including application of PGD2.

International Patent Publication No. WO 2011/014588 A2 describes topical formulations, such as those comprising a DP-2 receptor antagonist compound (e.g., ramatroban, ACT-129968 (setipiprant)), for treating a dermatological disease or condition (i.e., an abnormal state of the epidermis, dermis, and/or subcutaneous tissues), including autoimmune diseases, proliferative diseases, contact with an allergan or other irritant, scarring, burns, cutaneous mucinoses, or inflammatory diseases or conditions.

Garza et al., 2010 (J. Invest Dermatol. 2010; 130(Suppl. 1): S103, Abstract 615, XP9188519) and Garza et al., 2012 (Sci Transl Med. 2012 March 21; 4(126): 126ra34. doi:10.1126/scitranslmed.3003122) disclose that prostaglandin D2 inhibits hair growth and is elevated in bald scalp of men with androgenetic alopecia.

Accordingly, there exists a need for identifying and characterizing agents that regulate hair follicle growth.

### SUMMARY OF THE INVENTION

The present invention provides a composition comprising a DP-2 antagonist for use in stimulating hair growth in a subject having androgenetic alopecia by topical administration, wherein the DP-2 antagonist is AM211.

In an embodiment, the subject is further administered finasteride or minoxidil.

Other features and advantages of the present invention will become apparent from the following detailed description examples and figures.

### BRIEF DESCRIPTION OF THE DRAWINGS

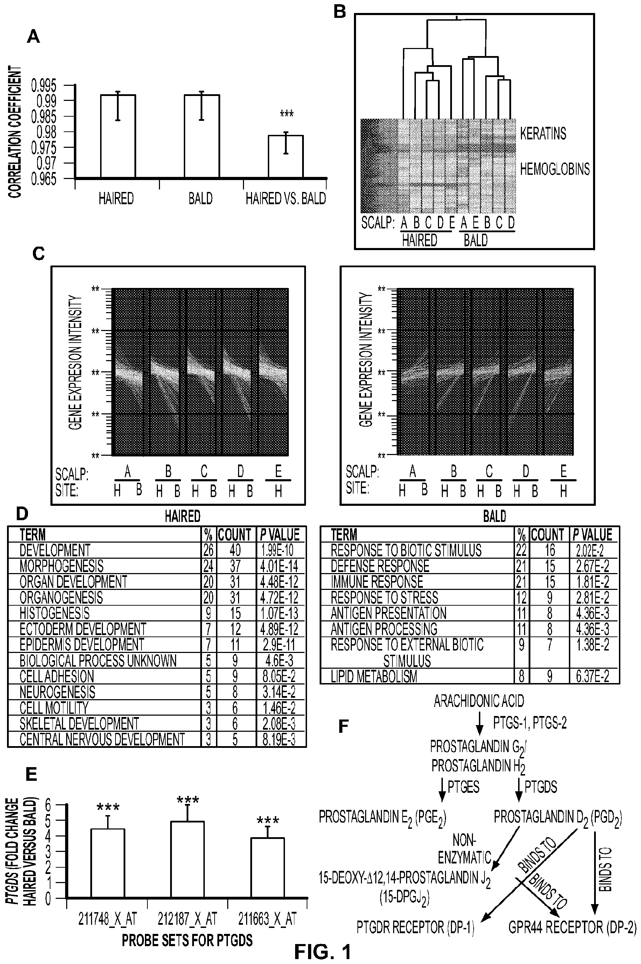

Fig. 1. Summary of gene expression profiles of haired versus bald scalp from five men with AGA. (A) Correlation coefficients to compare the degree of difference between all haired samples, between all bald samples, or between haired versus bald samples within individuals. Data are means ± SEM (n = 5). ***P < 0.001 compared to both hair only and bald only. (B) Gene clustering algorithm of 250 significant genes in haired and bald scalps in five men labeled A to E. (C) Discontinuous graphs with gene expression level on the y axis and samples grouped as pairs along the x axis for the 250 most significant genes divided into those higher in the haired (H) scalp (169; left) and those higher in the bald (B) scalp (81; right). In each pair, the first sample (beginning of line) is the haired scalp and the second sample (end of line) is the bald scalp. (D) Gene ontology categories in haired scalp (left) versus balding scalp (right). Percentages listed are numbers of transcripts in each category out of total number of unique gene transcripts. P values are modified Fisher exact EASE (Expression Analysis Systematic Explorer) score for the significant enrichment of each category of function. (E) Fold change in PTGDS expression in bald scalp compared to haired scalp. Data are means ± SEM (n = 5). ***P < 0.0001 between haired and bald scalp for each probe set, which covers distinct areas within the PTGDS gene. (F) Schematic of the PGD2 pathway.

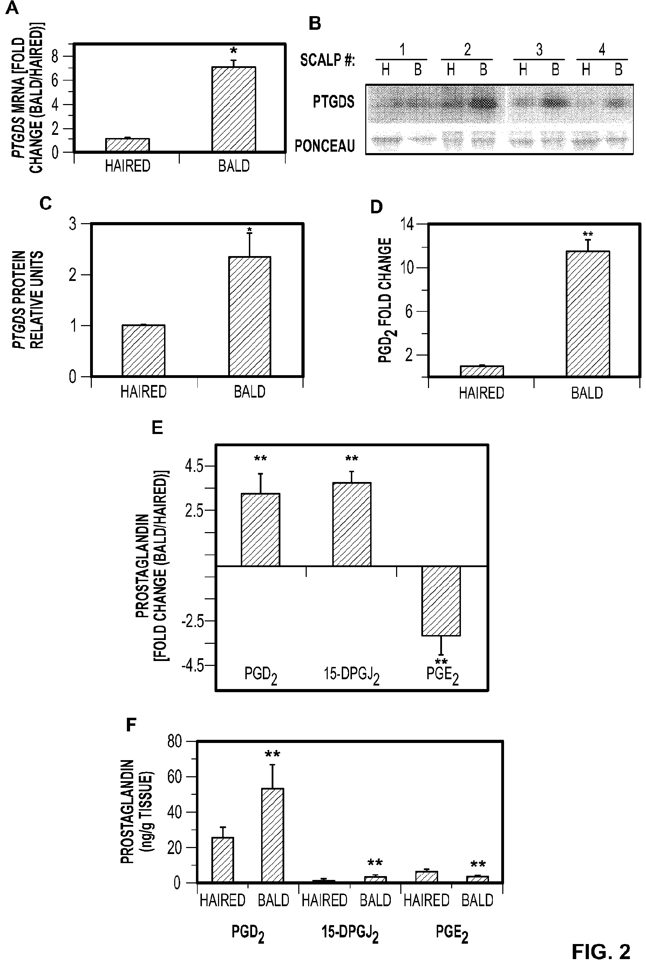

Fig. 2. Increased PGD2 pathway activity in balding scalp of men with AGA. (A) Expression of lipocalin-type PTGDS mRNA in bald versus haired scalp, as tested by qPCR. Data are means ± SEM (n = 4). (B and C) The amount of PTGDS protein in paired bald (B) and haired (H) scalps (n = 4), as shown by Western blotting with Ponceau stain used for verification of equal loading (B) and its quantitation as normalized to haired scalp (C). Data are means ± SEM. (D) PGD2 production in bald scalp, as tested by ELISA. Data are means ± SEM (n = 3). (E and F) Fold change in PGD2 (n = 17), 15-dPGJ2) (n = 7), and PGE2 (n = 17) expression in bald scalp compared to haired scalp (E). Total prostaglandin content is quantified in (F). Data are means ± SEM. *P < 0.05; **P < 0.01. In (F), P value compares haired versus bald samples for each prostaglandin.

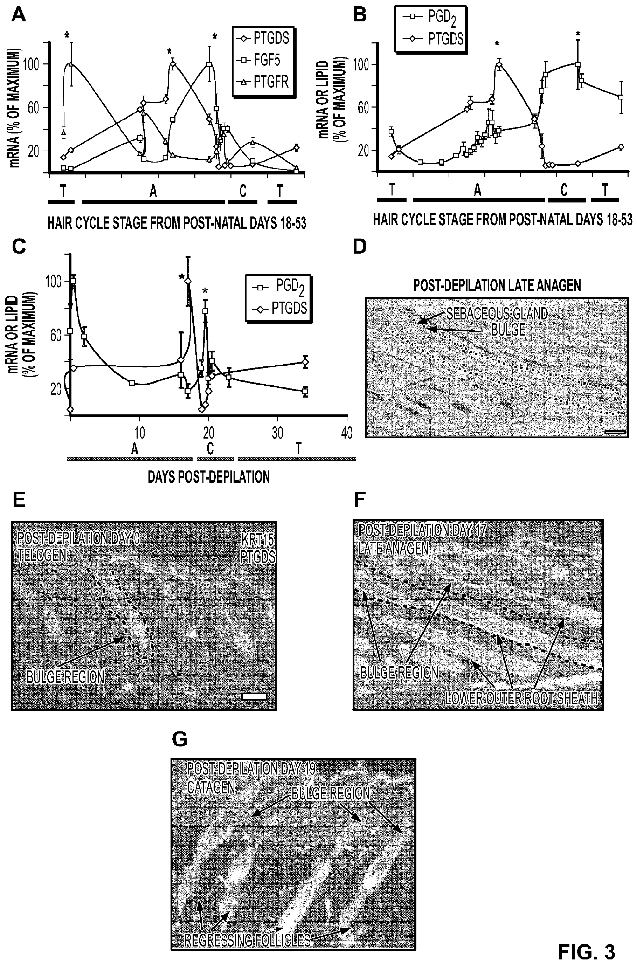

Fig. 3. An increase in Ptgds expression precedes elevation of PGD2 levels in the murine hair follicle during catagen. (A and B) At PN18 to PN53, Ptgds (n = 4), Ptgfr (n = 4), and Fgf5 (n = 4)mRNA (A and B) and PGD2 lipid (n = 3) (B) were measured at different phases of hair follicle cycling. Letters correspond to hair cycle stages: A, anagen; C, catagen; T, telogen. Data are means ± SEM. In (A), **P < 0.01 for Ptgds and Fgf5 late anagen versus first telogen and for Ptgfr first telogen versus second telogen. In (B), *P < 0.05 for PGD2 catagen versus early anagen. (C) Expression of Ptgds (n = 3) and production of PGD2 (n = 3) in murine skin during the depilation-induced hair follicle cycle. Data are means ± SEM. *P < 0.05 for Ptgds on day 17 versus day 0; **P < 0.01 for PGD2 day 19.5 versus day 37. (D) Murine outer root sheath keratinocytes below the stem cell-rich bulge area were stained for Ptgds (brown) 17 days after depilation. Dotted line delineates a full hair follicle. Scale bar, 100 mm. (E to G) Krt15 (red) and Ptgds (green) in permanent and nonpermanent compartments of the hair follicle on day 0 (anagen) (E), day 17 (late anagen) (F), and day 19 (catagen) (G) after depilation. Scale bars, 100 mm.

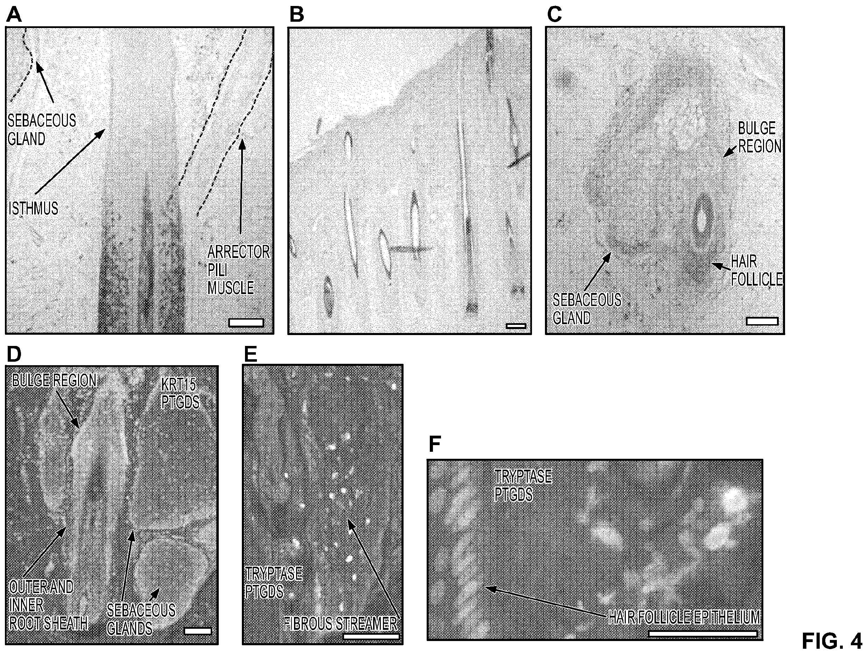

Fig. 4. PTGDS is expressed in the nonpermanent portion of select human hair follicles in haired scalp, and more variably in bald scalp. (A to C) PTGDS immunostaining (brown) with blue nuclear counterstain in normal human hair follicles (A and B) and bald scalp hair follicle (C). (A) A single follicle in haired scalp with staining for PTGDS inferior to the isthmus. (B) Most anagen follicles of haired scalp have little staining for PTGDS. (C) Miniaturized hair follicle with PTGDS staining in sebocytes and hair follicle keratinocytes. Scale bars, 100 mm. (D to F) Immunofluorescence staining for PTGDS (green), nuclei (blue), and either KRT15 (red) (D) or tryptase (red) (E and F) in the hair follicle and adjoining sebaceous gland in bald scalp. Catagen follicle with some PTGDS-positive cells also expressed the mast cell marker tryptase (E). Perifollicular cells show distinct populations of PTGDS- and tryptasepositive cells with overlapping expression (F). Scale bars, 100 mm.

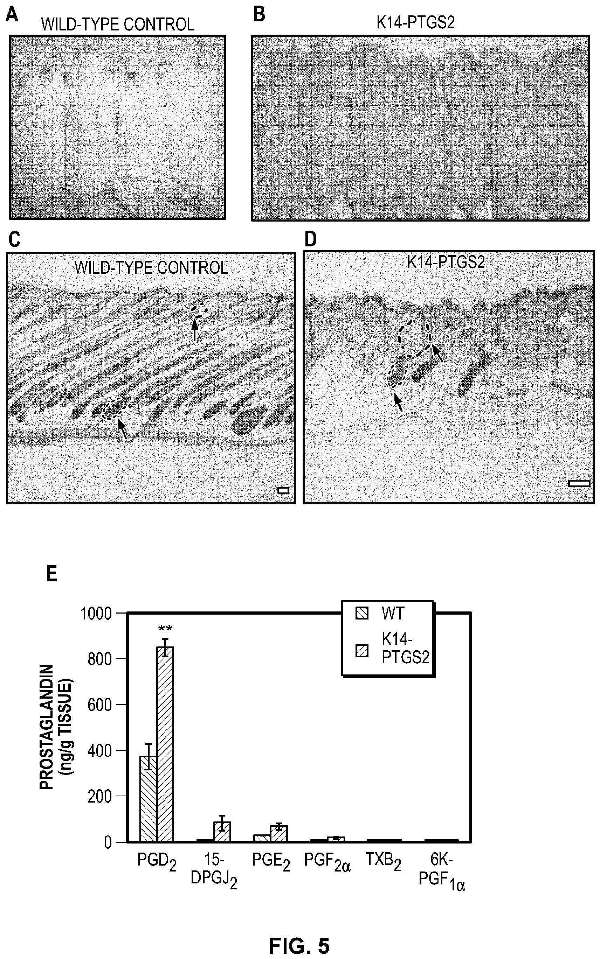

Fig. 5. K14-Ptgs2 transgenic mouse skin phenocopies AGA with alopecia, sebaceous gland hyperplasia, and elevated PGD2 levels in skin. (A and B) K14-Ptgs2 animals develop alopecia (B) compared to wild-type (WT) controls (A). (C and D) Hematoxylin and eosin-stained skin fromWT (C) and K14-Ptgs2 animals (D) shows sebaceous gland (yellow arrow) and hair follicle (black arrow) morphology. Scale bars, 100 mm. (E) Amount of different prostaglandins present in skin tissue fromWTmice (n = 5) and K14-Ptgs2 transgenic mice (n= 3).Data aremeans ± SEM. **P < 0.01 comparing WT to K14-Ptgs2.

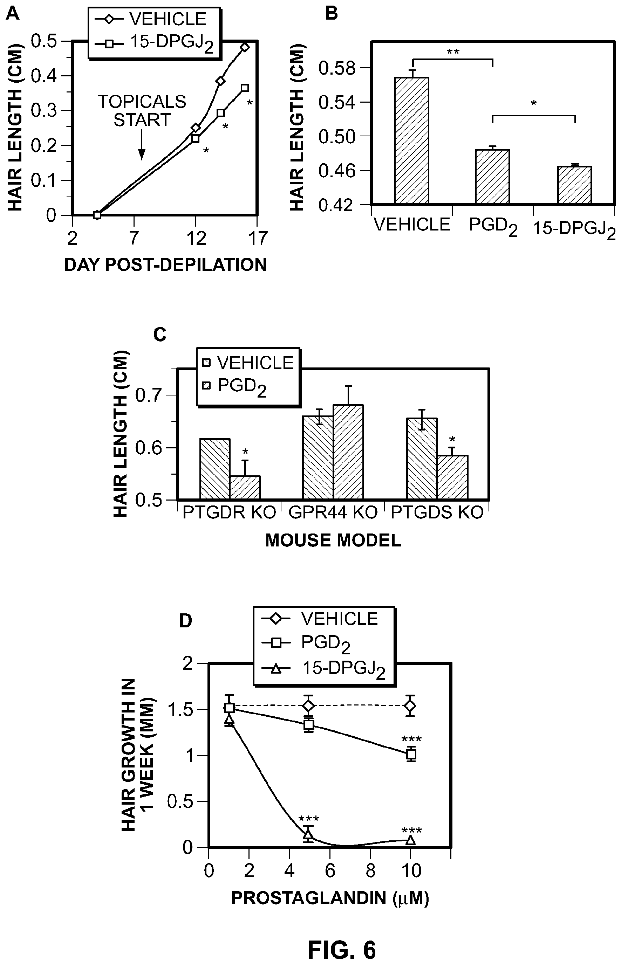

Fig. 6. PGD2 inhibits mouse and human hair growth through GPR44. (A) Hair growth over the course of 16 days after depilation. 15-dPGJ2 (10 mg) or vehicle was applied topically to mouse skin starting on day 8 after depilation. Data are means ± SEM (n = 3 per treatment group). *P < 0.05 compared to control. (B) Hair length 10 days after topical PGD2 (1 mg; n = 3), 15-dPGJ2 (1 mg; n = 3), or vehicle (n = 3) treatment. Data are means ± SEM. *P < 0.05; **P < 0.01. (C) Hair growth in Ptgdr (n = 8), Ptgds (n = 6), and Gpr44 (n = 11) knockout (KO) mice in response to PGD2. Data are means ± SEM and are representative of three independent experiments per mouse. *P < 0.05 compared to vehicle. (D) In vitro growth of explanted human hair follicles over the course of 7 days in culture with PGD2 (n = 3), 15-dPGJ2 (n = 3), or vehicle (n = 3). Data are means ± SEM. ***P < 0.001 compared to vehicle.

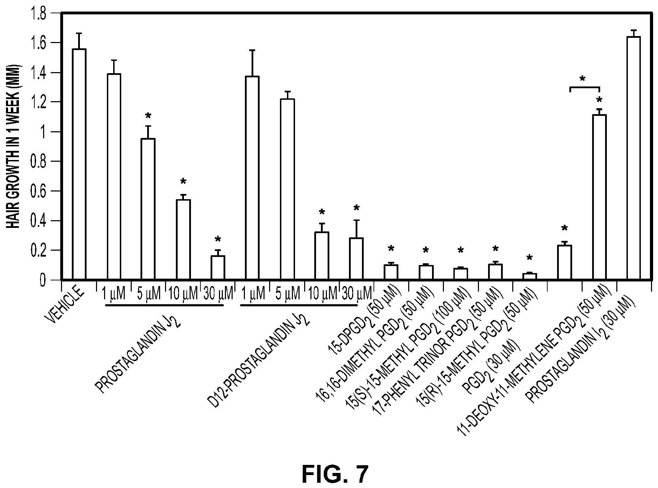

Fig. 7 Analogs of PGD2 inhibit human hair lengthening according to their relative agonism of GPR44. *In vitro* growth of explanted human hair follicles over the course of 7 days in culture with listed PGD2 analogues, prostaglandin I2, or vehicle control (n = 3 per treatment group). *P < 0.05 compared to vehicle, unless otherwise noted, Student's t test.

### DETAILED DESCRIPTION OF THE INVENTION

The invention relates to regulating hair growth by regulating the activity one of the prostaglandin D2 (PGD2) receptors, DP-2 (GPR44).

The present invention thus provides a composition comprising a DP-2 antagonist for use in stimulating hair growth in a subject having androgenetic alopecia by topical administration, wherein the DP-2 antagonist is AM211.

In an embodiment, the subject is further administered finasteride or minoxidil.

The present inventors surprisingly and unexpectedly found elevated levels of prostaglandin D2 synthase (PTGDS) at the message and protein levels in balding versus haired scalp from men with AGA. Given prostaglandin metabolism in the human hair follicle, the present inventors focused on the synthase, both in mouse (Ptgds) and in human (PTGDS). The enzymatic product of PTGDS, prostaglandin D2 (PGD2), was also found to be elevated in bald human scalp tissue. The present inventors show a close temporal relationship between elevations in both Ptgds mRNA and PGD2 levels in mice with hair follicle regression during normal hair follicle cycling. The present inventors further provide functional data showing that PGD2 and its nonenzymatic metabolite, 15-deoxy-D12,14-prostaglandin J2 (15-dPGJ2), inhibit hair growth in both mouse and human hair follicles. In mice and humans, the PGD2-mediated inhibition of hair growth required the G protein (heterotrimeric guanine nucleotide-binding protein)-coupled receptor 44 (GPR44 or DP-2), but not the prostaglandin D2 receptor 1 (Ptgdr or DP-1). Additionally, the present inventors present a mouse model (K14-Ptgs2) with elevated PGD2 levels in the skin that phenocopies human AGA. These results demonstrate, among other things, the role of PGD2 and DP-2 in the pathogenesis of AGA for hair treatment.

Also disclosed herein are methods for screening a compound as a potential agent for treating androgenetic alopecia, the methods comprising the steps of: measuring DP-2 activity in the presence of a compound suspected of being a potential agent for treating androgenetic alopecia and in the presence of prostaglandin D2; under the same conditions, measuring DP-2 activity in the absence of the compound suspected of being a potential agent for treating androgenetic alopecia and in the presence of prostaglandin D2, wherein a lower activity of DP-2 in the presence of said compound than in the absence of said compound is indicative of a potential agent for treating androgenetic alopecia. The methods may further comprise: measuring DP-1 activity in the presence of the compound suspected of being a potential agent for treating androgenetic alopecia and in the presence of prostaglandin D2; under the same conditions, measuring DP-1 activity in the absence of the compound suspected of being a potential agent for treating androgenetic alopecia and in the presence of prostaglandin D2, wherein a lower activity of DP-2 and an activity of DP-1 that is about equal in the presence and absence of said compound is indicative of a potential agent for treating androgenetic alopecia. A plurality of compounds may be screened.

Disclosed herein are methods for screening a compound as a potential agent for stimulating hair growth, the methods comprising the steps of: measuring DP-2 activity in the presence of a compound suspected of being a potential agent for stimulating hair growth and in the presence of prostaglandin D2; under the same conditions, measuring DP-2 activity in the absence of the compound suspected of being a potential agent for stimulating hair growth in the presence of prostaglandin D2, wherein a lower activity of DP-2 in the presence of said compound than in the absence of said compound is indicative of a potential agent for stimulating hair growth. The methods may further comprise: measuring DP-1 activity in the presence of the compound suspected of being a potential agent for stimulating hair growth and in the presence of prostaglandin D2; under the same conditions, measuring DP-1 activity in the absence of the compound suspected of being a potential agent for stimulating hair growth and in the presence of prostaglandin D2, wherein a lower activity of DP-2 and an activity of DP-1 that is about equal in the presence and absence of said compound is indicative of a potential agent for stimulating hair growth. A plurality of compounds may be screened.

As used herein, a "DP-2 agonist" is a molecule that is able to increase the amount or activity of DP-2, either within a cell or within a physiological system. For example, a DP-2 agonist is a substance that can bind to DP-2 selectively and elicit a physiological action of the DP-2 receptor.

As used herein, a "DP-2 antagonist" is a molecule that is able to decrease the amount or activity of DP-2, either within a cell or within a physiological system. For example, a DP-2 antagonist is a substance that can bind selectively to DP-2 receptor and inhibit the physiological action of the DP-2 receptor.

DP-2 antagonists are known in the art. Examples of some DP-2 antagonists are shown in Table 2:

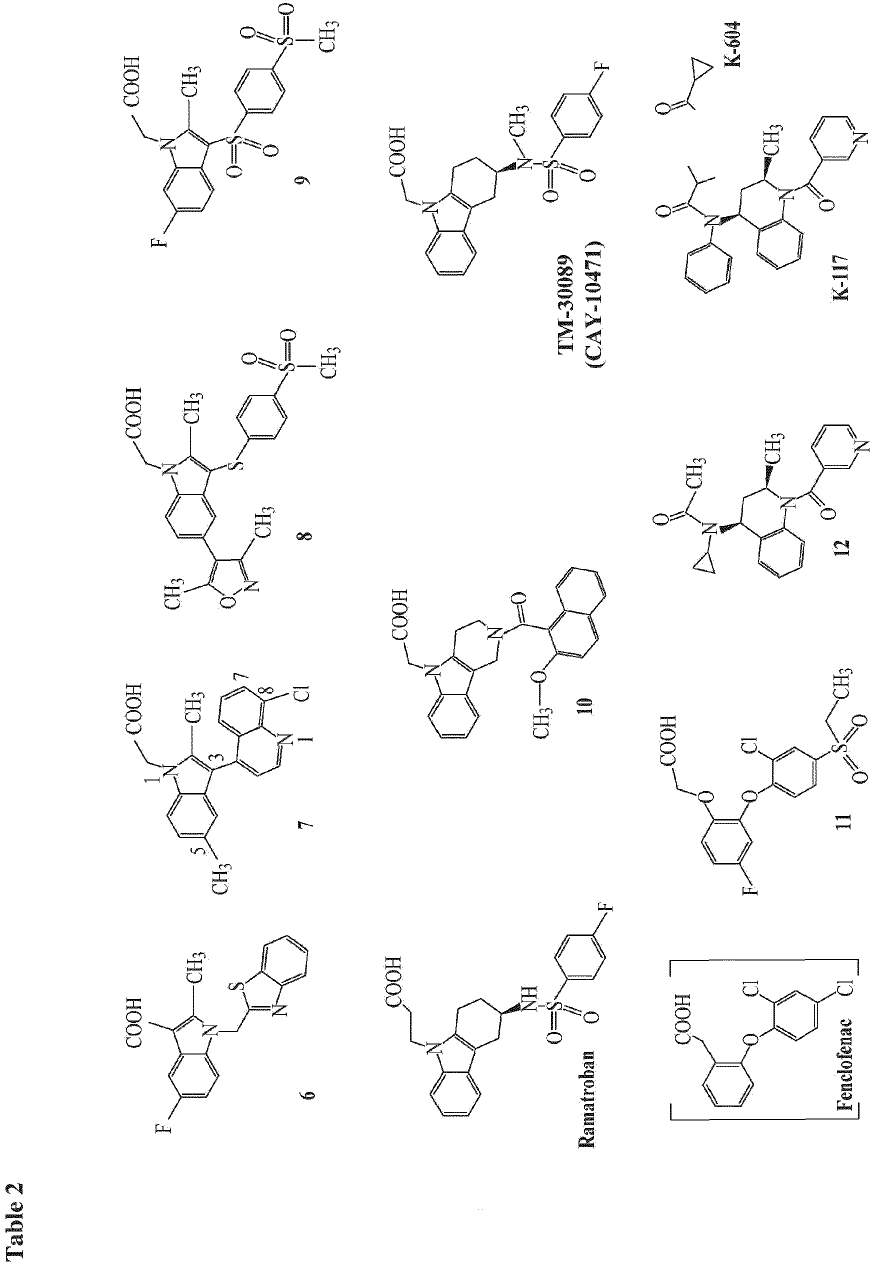

DP-2 antagonists can be divided into a number of classes. One class is Ramatroban and its analogues, examples of which are shown in Tables 2 and 3 (TM30642 - 3-{3-[(4-fluoro-benzenesulfonyl)-methyl-amino]-1,2,3,4-tetrahydro-carbazol-9-yl}-propionic acid; TM30643 - [3-(4-fluoro-benzenesulfo-nylamino)-1,2,3,4-tetrahydro-carbazol-9-yl]-acetic acid;TM30089 - {3-[(4-fluoro-benzenesulfonyl)-methyl-amino]-1,2,3,4-tetrahydro-carbazol-9-yl}-acetic acid.

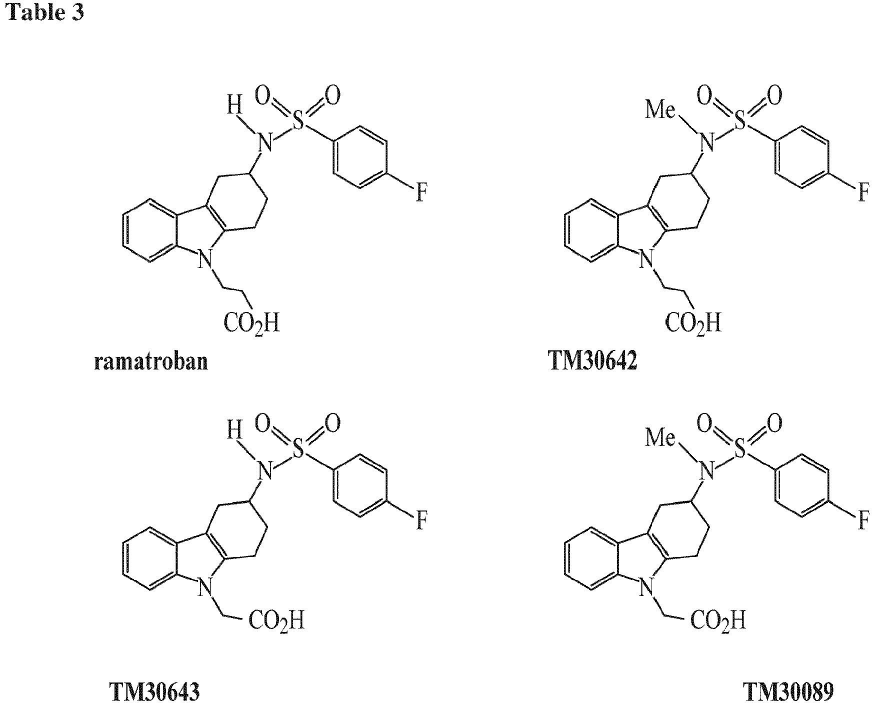

Another class of DP-2 antagonists are indole acetic acid derivatives. Examples of this class of compounds are compounds **6 - 9** of Table 2.

Another class of DP-2 antagonists are phenyl acetic acid derivatives. An example of this class of compounds, based on fenclofenac is compound **11** of Table 2.

Yet another class of DP-2 antagonists are tetrahyrdroquinoline derivatives. Examples of this class of compounds are compounds **12,** K117 and K-104 of Table 2.

Certain other DP-2 antagonists are the DP-2 antagonists that have been the subject of at least one clinical trial. Examples of such antagonists that have been the subject of at least one clinical trial include, but are not limited to, the DP-2 antagonists listed in Table 4 below:

**Table 4 Clinical Stage DP-2 Antagonists**

| *Company* | *Compound ID* | *Status* | *Reference^{a}* |
|---|---|---|---|
| Actelion | ACT-129968 (Setipiprant) | Phase IIb | NCT01225315 |
| Actimis | AP768 | Phase I | |
| Amira | AM211 | Phase I | |
| Amira | AM461 | Phase I | |
| Amgen | AMG853 | Discontinued after Phase II (2011) | NCT01018550 |
| Array BioPharma | ARRY-502 | Phase I | NCT01349725 |
| AstraZeneca | AZD1981 | Phase II | NCT01197794 |
| AstraZeneca | AZD8075 | Discontinued after Phase I (2010) | NCT00787072 |
| AstraZeneca | AZD5985 | Discontinued after Phase I (2010) | NCT00967356 |
| Merck | MK-7246) | Phase I | |
| Novartis | QAV680 | Phase II completed | NCT00814216 |
| Oxagen | OC000459 | Phase IIb | NCT01057927 |
| Oxagen | OC002417 | Back-up candidate | |
| Pulmagen | ADC3680B | Phase I | NCT01173770 |
| Shionogi | S-555739 | Phase IIb (Japan) | |

| | | | |
|---|---|---|---|
| ^{a}NCT numbers can be searched at the website: www.clinicaltrials.gov | | | |

For use in this invention, however, the DP-2 antagonist is AM211.

The structure of the DP-2 antagonist Ramatroban is provided below:

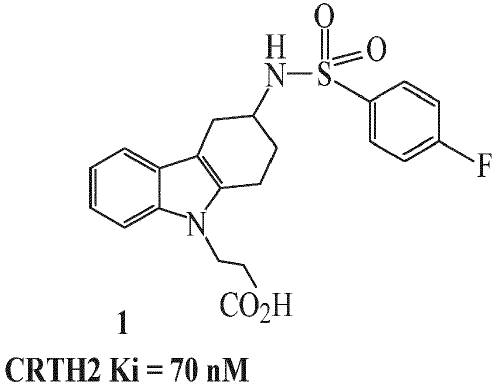

The structure of another DP-2 antagonist is provided below:

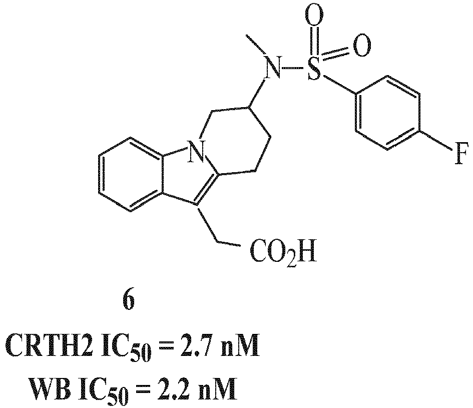

The structure of the DP-2 antagonist Setipiprant is provided below:

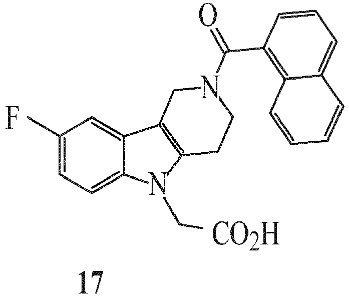

The structure of another DP-2 antagonist is provided below:

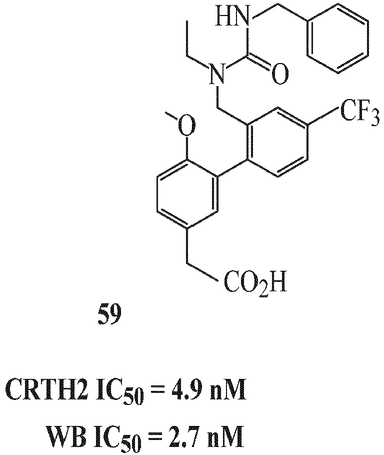

The structure of the DP-2 antagonist AZD1981 is provided below:

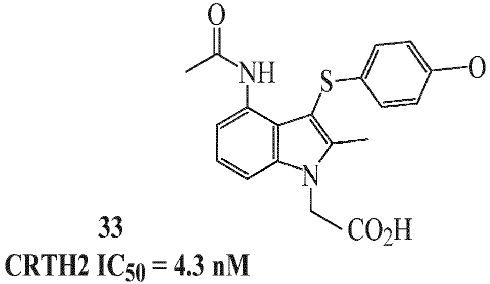

The structures of other DP-2 antagonists are provided below: 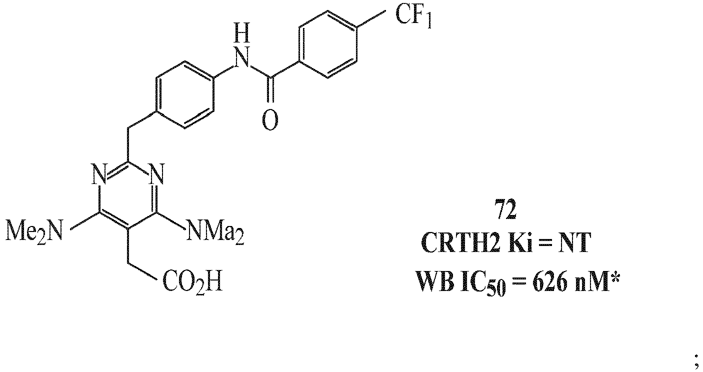 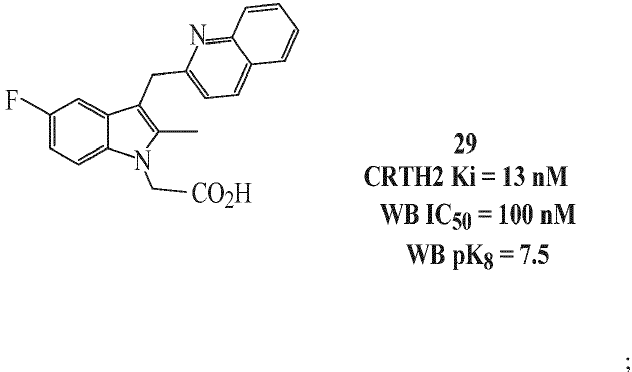 and

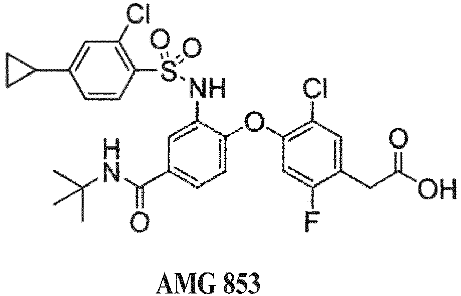

Additional DP-2 antagonists can be found in the following publications: EP1,170,594, EP1,435,356 WO2003/066046, WO2003/066047, WO2003/097042, WO2003/101961, WO2003/101981, WO2004/007451, WO2004/032848, WO2004/035543, WO2004/106302, WO2005/019171, WO2005/054232, WO2005/018529, WO2005/040112, GB2,407,318, WO2005/040114, WO2005/044260, WO2005/095397, WO2005/100321, WO2005/102338, WO2006/095183, WO2007/107772, US7,405,215.

Thus, provided herein are compositions for use in stimulating hair growth in a subject having androgenetic alopecia, the compositions comprising a DP-2 antagonist in an amount effective to enhance hair growth in the subject, wherein the DP-2 antagonist is AM211.

The composition may be a pharmaceutical composition comprising the DP-2 antagonist AM211 and at least one pharmaceutically acceptable excipient or carrier.

Provided herein are pharmaceutical compositions comprising the DP-2 antagonist for use in this invention and one or more pharmaceutically acceptable carriers. "Pharmaceutically acceptable carriers" include any excipient which is nontoxic to the cell or subject being exposed thereto at the dosages and concentrations employed. The pharmaceutical composition may include one or additional therapeutic agents.

Pharmaceutically acceptable carriers include solvents, dispersion media, buffers, coatings, antibacterial and antifungal agents, wetting agents, preservatives, buggers, chelating agents, antioxidants, isotonic agents and absorption delaying agents.

Pharmaceutically acceptable carriers include water; saline; phosphate buffered saline; dextrose; glycerol; alcohols such as ethanol and isopropanol; phosphate, citrate and other organic acids; ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; EDTA; salt forming counterions such as sodium; and/or nonionic surfactants such as TWEEN, polyethylene glycol (PEG), and PLURONICS; isotonic agents such as sugars, polyalcohols such as mannitol and sorbitol, and sodium chloride; as well as combinations thereof. Antibacterial and antifungal agents include parabens, chlorobutanol, phenol, ascorbic acid, and thimerosal.

The pharmaceutical compositions for use in the invention may be formulated in a variety of ways, including for example, solid, semi-solid (e.g., cream, ointment, and gel), and liquid dosage forms, such as liquid solutions (*e*.*g*., topical lotion or spray), dispersions or suspensions, powders, and liposomes, in a form suitable for topical administration. The composition may be formulated as an immediate, controlled, extended or delayed release composition.

More particularly, pharmaceutical compositions suitable for use include sterile aqueous solutions (where water soluble) or dispersions and sterile powders for the extemporaneous preparation of sterile solutions or dispersions. It should be stable under the conditions of manufacture and storage and will preferably be preserved against the contaminating action of microorganisms, such as bacteria and fungi. The carrier can be a solvent or dispersion medium containing, for example, water, ethanol, polyol (*e.g.,* glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Suitable formulations for use in the therapeutic methods disclosed herein are described in Remington's Pharmaceutical Sciences, Mack Publishing Co., 16th ed. (1980).

In some embodiments, the composition includes isotonic agents, for example, sugars, polyalcohols, such as mannitol, sorbitol, or sodium chloride.

Sterile solutions can be prepared by incorporating the molecule, by itself or in combination with other active agents, in the required amount in an appropriate solvent with one or a combination of ingredients enumerated herein, as required, followed by filtered sterilization. Generally, dispersions are prepared by incorporating the active compound into a sterile vehicle, which contains a basic dispersion medium and the required other ingredients from those enumerated above. Further, the preparations may be packaged and sold in the form of a kit such as those described in US Appl. Publ. No. 2002/0102208 A1. Such articles of manufacture will preferably have labels or package inserts indicating that the associated compositions are useful for treating a subject suffering from AGA.

Effective doses of the compositions for use in the present invention, as described herein vary depending upon many different factors, including means of administration, target site, physiological state of the patient, whether the patient is human or an animal, other medications administered, and whether treatment is prophylactic or therapeutic. Usually, the patient is a human but non-human mammals including transgenic mammals can also be treated. Treatment dosages may be titrated using routine methods known to those of skill in the art to optimize safety and efficacy.

The pharmaceutical compositions of the invention may include a "therapeutically effective amount." A "therapeutically effective amount" refers to an amount effective, at dosages and for periods of time necessary, to achieve the desired therapeutic result. A therapeutically effective amount of a molecule may vary according to factors such as the disease state, age, sex, and weight of the individual, and the ability of the molecule to elicit a desired response in the individual. A therapeutically effective amount is also one in which any toxic or detrimental effects of the molecule are outweighed by the therapeutically beneficial effects.

As used herein, the term, "selective" with respect to inhibition or stimulation means preferential inhibition or stimulation, respectively, of a first activity relative to a second activity (e.g., preferential inhibition of one pathway to another pathway; preferential inhibition relative to other receptors; or preferential inhibition of a mutant to a wild-type or vice versa). In some embodiments, the inhibitor is greater than five times more selective, greater than ten times more selective, greater than fifty times more selective, greater than 100 times more selective, or greater than 1000 times more selective for the desired molecular target or pathway versus an undesired molecular target or pathway. In some embodiments, an antagonist will inhibit the first activity of the molecular target or pathway by at least 2-fold, at least 5-fold, at least 10-fold, at least 20-fold, at least 50-fold, at least 100-fold relative to the second activity under the same conditions. It will be appreciated that in preferred embodiments, the DP-2 antagonist will be selective with respect to DP-1 by any of the foregoing amounts. The activity of a molecular target or pathway may be measured by any reproducible means. The activity of a molecular target or pathway may be measured *in vitro* or *in vivo.*

As used herein, "modulating" refers to "stimulating" or "inhibiting" an activity of a molecular target or pathway. For example, a composition modulates the activity of a molecular target or pathway if it stimulates or inhibits the activity of the molecular target or pathway by at least 10%, by at least about 20%, by at least about 25%, by at least about 30%, by at least about 40%, by at least about 50%, by at least about 60%, by at least about 70%, by at least about 75%, by at least about 80%, by at least about 90%, by at least about 95%, by at least about 98%, or by about 99% or more relative to the activity of the molecular target or pathway under the same conditions but lacking only the presence of the composition. In another example, a composition modulates the activity of a molecular target or pathway if it stimulates or inhibits the activity of the molecular target or pathway by at least 2-fold, at least 5 -fold, at least 10-fold, at least 20-fold, at least 50-fold, at least 100-fold relative to the activity of the molecular target or pathway under the same conditions but lacking only the presence of the composition. The activity of a molecular target or pathway may be measured by any reproducible means. The activity of a molecular target or pathway may be measured *in vitro* or *in vivo.* For example, the activity of a molecular target or pathway may be measured *in vitro* or *in vivo* by an appropriate assay known in the art measuring the activity. Control samples (untreated with the composition) can be assigned a relative activity value of 100%. A change in activity caused by the composition can be measured in the assays.

It will be appreciated that many of the antagonists described herein are strong inhibitors of their targets. For example, an antagonist may have a binding inhibitory activity (IC₅₀ value) for its desired molecular target (i.e., DP-2) of 1000 µM or less, 1000 nM or less, 100 nM or less, 10 nM or less, or especially 1 nM or less. In another example, the inhibitor has a binding inhibitory activity (IC₅₀ value) for its desired molecular target of between 1000 µM and 1 nM, between 1000 µM and 10 nM, between 1000 µM and 100 nM, between 1000 µM and 1000 nM, between 1000 nM and 1 nM, between 1000 nM and 10 nM, between 1000 nM and 100 nM, between 100 nM and 10 nM, between 100 nM and 1 nM, or between 10 nM and 1 nM.

In some embodiments, the antagonists disclosed herein inhibit their molecular targets or pathways by at least about 10%, by at least about 20%, by at least about 25%, by at least about 30%, by at least about 40%, by at least about 50%, by at least about 60%, by at least about 70%, by at least about 75%, by at least about 80%, by at least about 90%, by at least about 95%, by at least about 98%, or by about 99% or more.

As used herein, the terms "treat" and "treatment" refer to therapeutic treatment, including prophylactic or preventative measures, wherein the object is to prevent or slow down (lessen) an undesired physiological change associated with a disease or condition. Beneficial or desired clinical results include, but are not limited to, alleviation of symptoms, diminishment of the extent of a disease or condition, stabilization of a disease or condition (*i.e.*, where the disease or condition does not worsen), delay or slowing of the progression of a disease or condition, amelioration or palliation of the disease or condition, and remission (whether partial or total) of the disease or condition, whether detectable or undetectable. "Treatment" can also mean prolonging survival as compared to expected survival if not receiving treatment. Those in need of treatment include those already with the disease or condition as well as those prone to having the disease or condition or those in which the disease or condition is to be prevented.

In one embodiment, treating hair loss refers to treating a disease or disorder comprising balding, which in this invention refers to treating androgenetic alopecia (AGA).

As used herein, "inhibiting hair growth" refers to preventing hair growth, inhibiting the rate of hair growth, delaying hair germination, lengthening the hair cycle, lengthening telogen, maximal hair length or reducing the maximal hair diameter.

The DP-2 antagonist AM211 may be administered alone, or in combination with one or more therapeutically effective agents or treatments. In one embodiment, the DP-2 antagonist may be administered alone, or in combination with one or more therapeutically effective hair promoting agents (e.g., finasteride, minoxidil, or both) or treatments. The other therapeutically effective agent may be conjugated to the DP-2 antagonist, incorporated into the same composition as the DP-2 antagonist, or may be administered as a separate composition. The other therapeutically agent or treatment may be administered prior to, during and/or after the administration of the DP-2 antagonist.

In one embodiment, DP-2 antagonist is co-administered with one or more hair promoting agents (e.g., finasteride, minoxidil, or a combination thereof). In another embodiment, DP-2 antagonist is administered independently from the administration of a hair promoting agent. In one embodiment, DP-2 antagonist is administered first, followed by the administration of a hair promoting agent. In another embodiment, a hair promoting agent is administered first, followed by the administration of DP-2 antagonist.

Other therapeutically effective agents / treatments for a combination therapy to enhance hair growth include, for example, but not limited to, transplantation surgery and removing dermis or epidermis.

The administration of the DP-2 antagonist with other agents and/or treatments may occur simultaneously, or separately, via the same or different route, at the same or different times. Dosage regimens may be adjusted to provide the optimum desired response (*e.g.,* a therapeutic or prophylactic response).

In one example, a single bolus may be administered. In another example, several divided doses may be administered over time. In yet another example, a dose may be proportionally reduced or increased as indicated by the exigencies of the therapeutic situation. Dosage unit form, as used herein, refers to physically discrete units suited as unitary dosages for treating mammalian subjects. Each unit may contain a predetermined quantity of active compound calculated to produce a desired therapeutic effect. In some embodiments, the dosage unit forms of the invention are dictated by and directly dependent on the unique characteristics of the active compound and the particular therapeutic or prophylactic effect to be achieved.

The composition for use in the invention may be administered only once, or it may be administered multiple times. For multiple dosages, the composition may be, for example, administered three times a day, twice a day, once a day, once every two days, twice a week, weekly, once every two weeks, or monthly.

As used herein, a compound "inhibits" an activity if the compound reduces the desired activity by at least 10% relative to the activity under the same conditions but lacking only the presence of the compound. The activity may be measured by any reproducible means. The activity may be measured *in vitro* or *in vivo.* In some embodiments, compounds used in the methods described herein inhibit activity by at least about 20%, by at least about 25%, by at least about 30%, by at least about 40%, by at least about 50%, by at least about 60%, by at least about 70%, by at least about 75%, by at least about 80%, by at least about 90%, by about 95%, by about 98%, or by about 99% or more.

It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

"Administration" to a subject is not limited to any particular delivery system but is for topical e.g. transdermal administration. Administration to a subject may occur in a single dose or in repeat administrations, and in any of a variety of physiologically acceptable salt forms, and/or with an acceptable pharmaceutical carrier and/or additive as part of a pharmaceutical composition (described earlier). Once again, physiologically acceptable salt forms and standard pharmaceutical formulation techniques are well known to persons skilled in the art (see, for example, Remington's Pharmaceutical Sciences, Mack Publishing Co.).

The term "subject" includes mammals, *e.g.,* humans, companion animals (*e.g.,* dogs, cats, birds, and the like), farm animals (*e*.*g*., cows, sheep, pigs, horses, fowl, and the like) and laboratory animals (*e*.*g*., rats, mice, guinea pigs, birds, and the like). In some embodiments, the subject is male human or a female human.

As used herein, the phrase "pharmaceutically acceptable" refers to those compounds, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically acceptable excipient" means an excipient that is useful in preparing a pharmaceutical composition that is generally safe, non-toxic and neither biologically nor otherwise undesirable, and includes an excipient that is acceptable for veterinary use as well as human pharmaceutical use. A "pharmaceutically acceptable excipient" as used herein includes both one and more than one such excipient.

The term "about" or "approximately" means within an acceptable error range for the particular value as determined by one of ordinary skill in the art, which will depend in part on how the value is measured or determined.

The following examples are presented in order to more fully illustrate the preferred embodiments of the invention. They should in no way be construed, however, as limiting the broad scope of the invention.

### EXAMPLES

### MATERIALS AND METHODS

### Human tissue samples

This study used only normally discarded human scalp obtained anonymously and was approved by Penn's Institutional Review Board office as an exempt protocol. Normally discarded human scalp was obtained during hair transplantation. Tissues originated from adult Caucasian males age 40 to 65 who were not taking finasteride or minoxidil. All analyses used unique scalp samples not used in other experiments. To calculate average fold differences between haired and bald scalp, we created ratios for each patient and then averaged them. Microarrays were performed with published methods. For hair-lengthening experiments, discarded tissue from face lifts, which contain terminal hair, was used.

### Animals

All animal protocols were approved by the University of Pennsylvania Institutional Animal Care and Use Committee. Wild-type C57BL/6J female mice were purchased from Jackson Laboratory under request for mice of identical weights and birth dates for time-course studies. K14-Cox2 transgenic mice and Ptgdr, Gpr44, and Ptgds knockout mice were obtained from original sources. To decrease effects of endogenous testosterone, we used only female mice. Depilation to synchronize the hair cycle was performed as described. If an animal was found to be in a transitional state on the day of biopsy, the later-stage sample was labeled to be 12 hours more advanced.

### Real-time qPCR

Mouse dorsal back skin was removed with scissors and forceps. Human tissue was cut with scissors. Tissue (25 to 35 mg) was solubilized in 300 mL of Qiagen RLT buffer. Human scalp and mouse tissues were homogenized with a tissue rotor, and RNA was collected and converted to complementary DNA (cDNA) per the manufacturer's instructions with the Fibrous RNA Extraction Kit (Qiagen) and the High-Capacity cDNA kit (Applied Biosystems). cDNA (50 ng) was used in real-time qPCRs with Fast Mix

### Quantitative RT-PCR Master

Mix and measurements on the Step One Plus System (Applied Biosystems). b-Actin and 18S RNA probe standards were found to be equivalent to one another and did not fluctuate between samples of equal loading amounts. Fold change was calculated with the DDCT method. Primers used were inventoried TaqMan primers (Applied Biosystems): PTGDS (human), Ptgds (mouse), Fgf5 (mouse), Ptgfr (mouse), b-actin (human), b-actin (mouse), and 18S (human and mouse).

### Western blot

Human scalp tissue was homogenized, and protein amount was quantified with a BCA kit (Pierce). Equal amounts for each discarded tissue lysate (15 to 35 mg) were loaded for SDS-polyacrylamide gel electrophoresis. Polyvinylidene difluoride blots were probed with mouse monoclonal anti-PTGDS (Cayman Chemical), followed by anti-mouse secondary antibody conjugated to HRP (Vector). The blot was developed with electrochemiluminescence (Amersham), exposed to autoradiography, and quantified with ImageJ (National Institutes of Health), all per the manufacturers' instructions. Blots were subsequently stained with Ponceau Red (Sigma) to verify equal loading between all lanes tested.

### PGD2 enzyme-linked immunosorbent assay

Human scalp was obtained from hair transplants to measure the amount of PGD2. Immediately after surgery, samples were placed at 4°C in Dulbecco's modified Eagle's medium with 10% fetal bovine serum (FBS) and antibiotics/antimycotics, or they were snapfrozen. Tissue sample weights varied from 0.14 to 0.94 g. All values were normalized to the starting tissue weight. Within 1 to 2 days, samples were flash-frozen in liquid nitrogen and stored at -70°C for at least 24 hours. After thawing, samples were diluted in 1 mL of acetone and homogenized. Samples were centrifuged at 5000g for 10 min, supernatant was removed, and a second extraction was performed on the pellet. The combined supernatants were dried in a SpeedVac centrifuge and resuspended in 100 mL of enzyme immunoassay buffer provided in the Cayman Chemical Prostaglandin D2-MOX ELISA (enzyme-linked immunosorbent assay) kit. Manufacturer's instructions were followed for determination of PGD2 concentration relative to a standard curve. Values were normalized to haired scalp.

### Ultra-high-performance liquid chromatography

Human tissue was prepared as above for ELISA. To maximize prostaglandin yield in mice, we snap-froze the samples and kept them below 4°C at all times. Tissue from K14-Ptgs2 mice and Friend virus B-type (FVB) strain-matched animal controls was obtained at day 24 of life. For mouse tissues, excised epidermis and dermis were snapfrozen in acetone, minced, and homogenized with an Omni TH 115V with metal tip in acetone for less than 2 min. Homogenized tissue was then centrifuged at 16,000g for 10 min, with the supernatant saved. The acetone was dried under a gentle stream of nitrogen gas, after which the sample was redissolved in 1 mL of 5% acetonitrile in water, acidified with 20 ml of formic acid, and applied to a conditioned Strata-X SPE cartridge (Phenomenex). The SPE protocol included preconditioning with 1 mL of H₂O followed by 1 mL of acetonitrile, sample application, washing with 1 mL of H₂O, drying by application of house vacuum for 15 min, and elution with 1 mL of 10% acetonitrile in ethyl acetate. The eluate was then dried with a stream of nitrogen, redissolved in 200 mL of 30% acetonitrile in H₂O, and filtered through 0.2-mm Nylon Spin-X filters (Corning) before 100 mL was injected into the instrument for UHPLC analysis.

The UHPLC consisted of an Accela solvent-delivery system (Thermo) and Hypersil GOLD C18, 200 mm × 1 mm, 1.9-mm particle-size columns (Thermo). The mobile phase consisted of water (solvent A) and acetonitrile/methanol (95:5, solvent B), both with 0.005% acetic acid adjusted to pH 5.7 using ammonium hydroxide. The mobile phase gradient started at 20% B and increased in a linear manner to 35% at 10 min, followed by a linear increase to 80% B at 20 min, and held at 80% B for 3 min. The flow rate was 350 ml/min.

### Mass spectrometry

A TSQ Quantum Ultra instrument (Thermo) equipped with a heated electrospray ionization (ESI) source and triple quadrupole analyzer was used. The ESI source used nitrogen for sheath and auxiliary gas, set to 70 and 5 arbitrary units, respectively. The mass spectrometer was operated in the negative ion mode with a capillary temperature of 350°C and a spray voltage of 0 kV. The source offset was set to 6 V. The analyzer was operated in the selected reaction monitoring mode. The transitions monitored for the first 16 min were as follows: mass/charge ratio (m/z) 351→271 (collision energy, 15 V) for endogenous PGD2 and PGE2, m/z 355→275 (15 V) for tetradeuterated PGD2 and PGE2, m/z 353→193 (24 V) for endogenous PGF2a, and m/z 357→197 (24 V) for tetradeuterated PGF2a. For the last 7 min, m/z 315→271 (15 V) for endogenous 15-dPGJ2 and m/z 319→275 (15 V) for the tetradeuterated analog were monitored. All eicosanoids were purchased from Cayman Chemical.

### Immunohistology and immunofluorescence

Mouse-back or human-scalp skin was fixed in 4% paraformaldehyde, embedded in parafilm, and sectioned at 5 to 10 mm. Non-immunoslides were stained for hematoxylin and eosin (Fisher Scientific). For immunostaining, polyclonal rabbit anti-PTGDS (Cayman Chemical) or monoclonal mouse anti-KRT15 (Lab Vision, clone LHKRT15) was applied followed by biotinylated anti-rabbit or anti-mouse, respectively. ABC kit (Vector labs) was used with horseradish peroxidase and diazoaminobenzene for color development. Methyl green (Vector Labs) was used to counterstain the tissue for immunohistochemistry; antigen staining appears brown and counterstain appears bluish green. Staining was visualized in a traditional light microscope (Nikon). Skin was treated with the same primary antibodies as described for immunohistochemistry. Anti-rabbit Alexa Fluor 488 (Invitrogen) and anti-mouse rhodamine (Vector Labs) were used as secondary antibodies. Tissues were subsequently mounted in 4',6-diamidino-2-phenylindole (DAPI) medium (Vector Labs). Staining was visualized in a traditional fluorescence microscope with red, green, and blue filter cubes (Leica).

### Keratinocyte cell culture

Human foreskin keratinocytes were isolated from neonatal foreskins. Equal densities of keratinocytes were plated (50,000 cells/cm2) between passages 2 and 3 after initiation into six-well culture dishes. Cells were maintained in 2 mL of EpiLife medium (Cascade Biologics) and treated for 12 to 48 hours with 100 nM PGD2, 1 mM PGE2, or 10 mM 15-dPGJ2 (Cayman Chemical) that had been dissolved in acetone. Unless specified otherwise, cells were treated with 10 mM prostaglandins for 20 hours. Acetone alone was used as a control.

### Flow cytometry

During culture, human keratinocytes were pulsed with 0.2 mM bromodeoxyuridine (BrdU) for the final 3 hours of incubation with prostaglandins. Cells were typsinized to release cells into suspension, counted, fixed, and permeabilized (Caltech). Cells were stained with 20 mL of anti-activated caspase 3 (Becton Dickinson, clone CPP32) or anti-BrdU fluorescein isothiocyanate (Becton Dickinson, clone B44) per 106 cells. Cells were resuspended in phosphate-buffered saline (PBS) with 5% FBS and DAPI (5 mg/mL) before being run on an LSRII flow cytometer (Becton Dickinson). Sub-G1 cells were gated as those containing less than diploid amounts of DNA.

### Mouse hair-lengthening studies

To compare the effect of vehicle, PGD2, and 15-dPGJ2 on the hair cycle among wild-type and knockout mice, we applied 1 mg of PGD2 in 200 mL of acetone or acetone alone to the central back on days 8, 10, 12, 14, 16, and 18 after depilation, with measurement of hair length performed on day 20. To test the time course of hair lengthening on 15-dPGJ2, we used 10 mg instead of the PGD2 above. Hair length was measured as described, with the selection of only awl (non-zigzag) hairs measured. For testing mutant mouse strains, the experiment was repeated on three separate litters.

### Human hair follicle culture

The hair follicle organ culture model was performed as described. Briefly, human hair follicles in growth phase (anagen) were isolated from face and brow-lift tissue obtained from plastic surgeons. At least three different human donors (a mixture of men and women) were used for each compound tested. All subjects were in the age range of 40 to 60 years. The skin was sliced into thin strips of 1 to 2 mm, exposing two to three rows of follicles that could readily be dissected. Follicles were placed into 0.5 mL of William's E medium (Life Technologies) supplemented with L-glutamine (2 mM), insulin (10 mg/ml), hydrocortisone (10 ng/ml), penicillin (100 U), streptomycin (0.1 mg/ml), and amphotericin B (0.25 mg/ml). The follicles were incubated in 24-well plates (one follicle per well) at 37°C in an atmosphere of 5% CO2 and 95% air. PGD2, PGD2 metabolites, and PGD2 analogs (Cayman Chemical) were dissolved into dimethyl sulfoxide (DMSO) as 100× stock solutions (for example, 0.5 mM for 5 mM treatments and 1 mM for 10 mM treatments). DMSO vehicle controls were used at 1% (v/v). Hair follicles were treated with DMSO only as a control. Follicles were imaged typically on day 0 (the day follicles were placed in culture) and again on day 7. The length of the hair fiber was assessed with Vision Builder (National Instruments). The growth of the hair fiber was calculated by subtracting the length on day 0 from that determined on day 7. Each measurement reported is the average of the results from 14 to 20 follicles.

### Statistical analysis

For all P value calculations, paired Student's t test with a one-tailed distribution was calculated and P < 0.05 was considered to be significant. All data are means ± SEM. Analysis of variance (ANOVA) test was used to rank genes from microarray as primarily different with respect to site (haired versus bald) and irrespective of person, date of biopsy, or date of microarray.

### RESULTS

### EXAMPLE 1

### PTGDS and PGD2 are elevated in bald scalp

To assess global gene expression changes in the bald scalp of men with AGA, we performed gene expression microarrays comparing bald to haired scalp in five men with AGA. Correlation coefficients among haired and among bald replicate samples approached 1 (Fig. 1A). Correlation coefficients comparing haired to bald scalp within individuals were also close to 1, indicating the advantage of paired samples as internal controls. We identified 250 transcripts that were differentially expressed in human haired versus bald scalp. As evidence for the validity of these transcripts, a clustering algorithm sorted out samples as either haired or bald, on the basis of the expression of these genes (Fig. 1B). Hair keratins were up-regulated in haired scalp, as would be expected. For unclear reasons, hemoglobin related transcripts were elevated in bald scalp. In haired scalp, 169 genes were elevated, and 81 genes were elevated in bald scalp, with the expected directions of expression (Fig. 1C). We determined enriched gene ontology functional classifications for these genes and identified overlapping categories. In the haired scalp, morphogenesis and developmental pathway genes were over-expressed, whereas in the bald scalp immune response genes were over-expressed (Fig. ID), consistent with the known histology of AGA.

Considering the clinical use of prostaglandin F2a (PGF2a)-related compounds to promote hair growth, and the often antagonistic functions of prostaglandins, we were intrigued to find PTGDS as one of the most highly expressed transcripts in human male balding scalp (Fig. 1, E and F). Of the three probe sets that covered PTGDS on the microarray, all were elevated in balding scalp (Fig. 1E). Increased PTGDS expression and PTGDS protein levels were further confirmed in balding versus haired human scalp by real-time quantitative polymerase chain reaction F2 (qPCR) (Fig. 2A) and Western blotting (Fig. 2, B and C). To better understand the role of PTGDS in balding, we measured levels of its synthase product, PGD2, in both balding and haired human scalps. We discovered a significant increase in PGD2 in the balding scalp compared to haired scalp by immunoassay (Fig. 2D). The absolute level of PGD2 was 16.3 ng/g tissue in balding scalp and 1.5 ng/g tissue in haired scalp. PGD2 levels were then measured using ultra-high-performance liquid chromatography mass spectrometry (UHPLC-MS) because of its reported superior accuracy in measuring prostaglandins compared to immunoassay. In a larger series of paired bald and haired samples from17 men with AGA, we noted an increase in PGD2 in bald scalp compared to haired scalp (Fig. 2E). Other prostaglandins have activity on hair follicle growth. We measured levels of additional prostaglandin species in balding and haired scalps. The nonenzymatic metabolite of PGD2, 15-dPGJ2, was also increased in balding scalp compared to haired scalp (Fig. 2E). However, the absolute level of 15-dPGJ2 (3.8 ng/g bald scalp, 1.3 ng/g haired scalp) was lower than PGD2 (53.5 ng/g bald scalp, 25.6 ng/g haired scalp) (Fig. 2F). UHPLC-MS did detect higher levels of PGD2 in balding scalp than immunoassay (53.5 versus 16.3 ng/g tissue), although both methods demonstrated increased PGD2 mass in bald scalp. In contrast to PGD2, the level of prostaglandin E2 (PGE2), which is synthesized by PTGES rather than PTGDS, was more abundant in haired scalp compared to balding scalp (Fig. 2E), with 3.1 ng/g balding scalp and 6.4 ng/g haired scalp as detected by UHPLC-MS (Fig. 2F).

### EXAMPLE 2

### Nonpermanent follicle mouse keratinocytes express Ptgds at the end of anagen

To better understand the normal biological function of Ptgds and PGD2 on hair growth, we capitalized on the well-characterized, synchronized hair cycle of the mouse. Fluctuations in gene expression and protein levels can be tracked to different stages of the hair follicle cycle by examining skin at different ages or after depilation of the mouse hair, which induces a synchronized new hair follicle cycle. In the process of investigating the features of the synchronized mouse hair cycle, we verified hair cycle stage relative to postnatal (PN) day in mouse skin biopsy samples. We confirmed that, as suggested by gross appearance, the histological features of mice at PN21 were those of late telogen, PN35 of late anagen, and PN46 of catagen. Ptgds mRNA, as measured by qPCR, peaked in the late stage of anagen and was sevenfold higher compared to the resting phase (telogen) at PN20 (Fig. 3A). Thus, Ptgds expression was lowest during telogen and increased progressively through anagen to peak at late anagen near the time of transition to catagen, the phase of regression. To more closely examine the proximity of the Ptgds expression peak to the catagen transition, we compared it to the expression of fibroblast growth factor 5 (Fgf5), a known marker of catagen onset. Fgf5 expression peaked in late anagen compared to telogen (Fig. 3A). Together, Fgf5 and Ptgds peak expression levels overlapped during late anagen, just before the onset of catagen. We next sought to identify control genes expressed differently from Fgf5 and Ptgds. As a marker of early anagen, we measured the expression of the receptor for PGF2a, which is known for its ability to induce hair growth both in mice and in humans. In contrast to the late peak of Ptgds and Fgf5, prostaglandin F receptor (Ptgfr) mRNA peaked in early anagen or late telogen, with a 21-fold change versus second telogen (PN52) (Fig. 3A) and a 3-fold change versus first telogen (PN20).

To determine the temporal relationship between expression of Ptgds and the production of PGD2, we measured PGD2 levels by HPLC-MS in identical mice from which mRNA data were determined in Fig. 3A. We discovered that PGD2 production peaked after the apex of Ptgds expression. During the catagen stage, at PN46, PGD2 reached a peak level of 87.6 ng/g tissue compared to a nadir in early anagen on PN24 of 7.4 ng/g tissue (Fig. 3B). Thus, Ptgds mRNA is first expressed in the skin in late anagen, and subsequently, PGD2 is produced via Ptgds protein during catagen. Although early hair follicle cycle stages in individual mice are naturally synchronized, animals of the same age and even litter can exhibit heterogeneity with respect to hair follicle cycling. Therefore, we repeated the above analysis using mice that had been depilated simultaneously to synchronize the hair cycle between animals as well as within animals. Corroborating our results from the spontaneous hair cycle, Ptgds mRNA also fluctuated with the hair cycle in depilated animals. Ptgds mRNA peaked in late anagen, followed by the peak of PGD2 in catagen (Fig. 3C). PGD2 production peaked on day 19.5 (catagen), with 199.9 ng/g tissue compared to a nadir of 27.6 ng/g tissue on day 37. We also noted a unique peak of PGD2 production within hours of depilation, which was not seen in the spontaneous hair cycle in Fig. 3B. This coincides with degranulation of mast cells observed previously after depilation. The more narrow peaks of markers in the depilated hair cycle likely reflect tighter synchronization of the hair follicle cycle after depilation. To better define expression of Ptgds, we performed immunohistochemistry on tissue sections from skin of the animals used for the time course in Fig. 3C. Ptgds was evident in late anagen in the keratinocytes of the outer root sheath below the stem cell-rich bulge area marked by the arrector pili muscle (Fig. 3D). This area of the follicle regresses during catagen.

To corroborate this pattern seen by immunohistochemistry, we next examined double immunofluorescence labeling for both Ptgds and keratin 15 (Krt15), a hair follicle stem cell marker. At day 0 of depilation (telogen), Krt15 was present in the bulge area at the most inferior permanent part of the hair follicle, which persists into telogen (Fig. 3E). We did not identify Ptgds, which was consistent with qPCR data showing lack of expression of Ptgds during telogen (Fig. 3A). Also consistent with qPCR, at day 17 (late anagen), we found a detectable Ptgds pattern by immunohistochemistry (Fig. 3, D and F). Lower outer root sheath cells-but not bulge cells-expressed Ptgds. Furthermore, at day 19 after depilation, keratinocytes in catagen follicles expressed Ptgds, without overlapping Krt15-positive keratinocytes (Fig. 3G). These results demonstrate that Ptgds is abundant in nonpermanent keratinocytes of the hair follicle in mouse.

### EXAMPLE 3

### PTGDS is expressed in nonpermanent keratinocytes of the human hair follicle

In normal terminal human hair follicles, we found a similar presence of PTGDS, primarily in the nonpermanent hair follicle below the arrector pili muscle (Fig. 4A). However, staining was variable with many normal human follicles exhibiting little or no staining (Fig. 4B). This is consistent with lower levels of PTGDS mRNA in haired versus bald scalp and likely also reflects the lack of synchronicity in human hair follicle cycling. In miniaturized hair follicles in balding human scalp, sebaceous gland and hair follicle keratinocytes outside of the bulge (Fig. 4, C and E), and in some cases within the suprabasal bulge (Fig. 4D), expressed PTGDS. We also detected PTGDS outside of the hair follicle epithelium, indicating potential sources of PGD2 in the dermis (Fig. 4, E and F). In hair follicles undergoing catagen, PTGDS was present in mast cells within the fibrous streamer, which is the former site of the regressed follicle (Fig. 4E). These results in mouse and human demonstrate that the lipocalin PTGDS and its product PGD2 are predominantly expressed in the transient portion of the follicle at a time when the follicle begins regressing. These findings are in line with the hypothesis that the PGD2 pathway inhibits hair follicle growth.

### EXAMPLE 4

### Transgenic mice overexpressing Ptgs2 in the epidermis phenocopy AGA

Given the correlation of increased levels of PGD2 with balding scalp in humans and the presumptive inhibitory role of PGD2 on the mouse follicle, we hypothesized that mice with high levels of PGD2 in the skin might develop features of AGA. Because Ptgs2 (cyclooxygenase 2, prostaglandin G/H synthase) is the enzyme upstream to Ptgds, we further hypothesized that mice overexpressing Ptgs2 would have elevated PGD2 levels. Transgenic mice that overexpress Ptgs2 in the epidermis had been developed previously for carcinogenesis studies. The hair follicles in these K14-Ptgs2 transgenic mice were noted to enter catagen prematurely, and these mice reportedly developed alopecia and sebaceous gland enlargement.

We further analyzed the skin and hair follicles of the K14-Ptgs2 mouse. These mice developed alopecia, which was evident as a decrease in the normal murine pelage coat compared to control (Fig. 5, F5 A and B). By histology, we also detected sebaceous gland hyperplasia as indicated by enlarged sebocytes clustered around the hair follicle (Fig. 5, C and D). The hair follicles in the K14-Ptgs2 mice were miniaturized compared to controls (Fig. 5, C and D), and these follicles bore a marked resemblance to the miniaturized follicles in human bald scalp. To determine the prostaglandin content in the alopecic skin of the K14-Ptgs2 mice, we measured prostaglandin levels by HPLC-MS during the anagen phase of the hair follicle cycle. PGE2 was elevated in the K14-Ptgs2 mice compared to age-matched wild-type controls, as was previously shown using immunoassays measuring PGE2 and PGF2a content in biopsied mouse skin. PGD2 was also elevated and was more abundant than PGE2 in both wildtype and K14-Ptgs2 mice. 15-dPGJ2 was elevated in K14-Ptgs2 mice compared to controls and demonstrated the largest fold increase (∼14-fold), although baseline values were low (5.7 ng/g tissue) (Fig. 5E). We found low levels (18.4 ng/g tissue) of PGF2a, and an absence of prostacyclin (6k-PGF1a) and thromboxane (TxB2) (Fig. 5E), which are not known to be present in normal skin. Together, the balding phenotype in these mice is likely a result of the overwhelming PGD2 and 15-dPGJ2 inhibitory effects on the hair follicle, despite the presence of PGE2, a known promoter of hair growth.

### EXAMPLE 5

### 15-dPGJ2 and PGD2 inhibit hair growth in mouse and human hair follicles

Given the temporal peak of PGD2 before the apoptotic catagen stage, and the ability of its metabolite 15-dPGJ2 to induce apoptosis in other cell types, we tested the effects of the prostaglandins on primary cell culture of keratinocytes isolated from neonatal foreskin. 15-dPGJ2 induces apoptosis, as evidenced by plasma membrane blebbing and cell retraction/shrinkage. 15-dPGJ2 also decreased cell density, cell division, and live-cell numbers. Perhaps because the origin of these keratinocytes was not the hair follicle, PGD2 had no such effect on the cells. However, 15-dPGJ2 did increase sub-Gl DNA quantities and activated caspase 3 in human keratinocytes, which are features of apoptotic cell death. We therefore hypothesized that at least 15-dPGJ2, if not also PGD2, could directly inhibit hair growth in vivo. 15-dPGJ2 was applied topically to dorsal back skin of C57BL/6 mice that had been depilated to synchronize the hair follicle cycle. Starting on day 8 after depilation and continuing every other day, we applied 10 mg of 15 dPGJ2 or acetone vehicle. Hair length was measured on days 4, 12, 14, and 16 after depilation. On days 12 to 16, hair at the site of treatment was shorter than in vehicle-treated animals F6 (Fig. 6A). To determine a minimal effective dose, we tested the application of 1 mg of both PGD2 and 15-dPGJ2 as above and measured hair length on day 20 after depilation. PGD2 inhibited hair growth, but to a lesser extent than 15-dPGJ2 (Fig. 6B).

We found no evidence of changes in hair follicle cycling grossly or by histologic examination. Having demonstrated PGD2-mediated inhibition of hair growth, we next sought to determine which receptor was responsible for this effect. The two canonical receptors for PGD2 are PTGDR (also known as DP-1) and GPR44 (DP-2). Both DP-1 and DP-2 have been reported to be expressed by outer root sheath keratinocytes in the hair follicle, among other sites. We therefore tested the capacity for PGD2 to inhibit hair growth in both Ptgdr null mice and Gpr44 null mice using Ptgds null mice as a control. Whereas Ptgds and Ptgdr knockout mice were both susceptible to the inhibition of hair lengthening, Gpr44 null mice were resistant to the inhibitory effect of PGD2 (Fig. 6C). These data show that GPR44, rather than PTGDR, is the receptor for PGD2- mediated inhibition of hair lengthening and could therefore be a therapeutic target for AGA.

To test the effect of PGD2 on human hair growth, we used explanted human hair follicles maintained in culture for 7 days. We added increasing amounts (from 0 to 10 mM) of PGD2, 15-dPGJ2, or vehicle to the culture medium and measured hair length (Fig. 6D). Starting at 5 mM, PGD2 and 15-dPGJ2 significantly inhibited hair growth. At 10 mM, PGD2-treated hair was 62 ± 5% shorter than vehicle, whereas 10 mM 15-dPGJ2 completely inhibited all hair growth. We tested a variety of other PGD2 analogs and found them to be capable of inhibiting hair lengthening. Agonism for GPR44 correlated with the ability to inhibit hair lengthening (Fig. 7). For example, the weak agonist 11-deoxy-11-methylene PGD2 had the lowest activity (hair growth inhibition) of the compounds tested. Note that GPR44 has nanomolar affinities for ligands, but as is typical for this type of experiment, higher concentrations were required to overcome tissue penetration and compound lysis. Thus, in both mouse and human, PGD2 and 15-dPGJ2 inhibit hair growth, likely through the GPR44 receptor.

We show that prostaglandin D2 synthase (PTGDS) is elevated at the mRNA and protein levels in bald scalp compared to haired scalp of men with AGA. The product of PTGDS enzyme activity, prostaglandin D2 (PGD2), is similarly elevated in bald scalp. During normal follicle cycling in mice, Ptgds and PGD2 levels increase immediately preceding the regression phase, suggesting an inhibitory effect on hair growth. We show that PGD2 inhibits hair growth in explanted human hair follicles and when applied topically to mice. Hair growth inhibition requires the PGD2 receptor G protein (heterotrimeric guanine nucleotide)-coupled receptor 44 (GPR44), but not the PGD2 receptor 1 (PTGDR). Furthermore, we find that a transgenic mouse, K14-Ptgs2, which targets prostaglandin-endoperoxide synthase 2 expression to the skin, demonstrates elevated levels of PGD2 in the skin and develops alopecia, follicular miniaturization, and sebaceous gland hyperplasia, which are all hallmarks of human AGA.

## Claims

1. A composition comprising a DP-2 antagonist for use in stimulating hair growth in a subject having androgenetic alopecia by topical administration, wherein the DP-2 antagonist is AM21 1.

2. The composition for use according to claim 1, wherein the subject is further administered finasteride or minoxidil.

## Patentansprüche

1. Zusammensetzung, die einen DP-2-Antagonisten umfasst, zur Verwendung beim Stimulieren von Haarwachstum bei einem Subjekt, das androgenetische Alopezie aufweist, durch topische Verabreichung, wobei der DP-2-Antagonist AM211 ist.

2. Zusammensetzung zur Verwendung nach Anspruch 1, wobei dem Subjekt ferner Finasterid oder Minoxidil verabreicht wird.

## Revendications

1. Composition comprenant un antagoniste de DP-2 destinée à être utilisée pour stimuler la croissance des cheveux chez un sujet atteint d'alopécie androgénétique par administration topique, l'antagoniste de DP-2 étant l'AM211.

2. Composition destinée à être utilisée selon la revendication 1, le sujet recevant en outre du finastéride ou du minoxidil.
